# FASCICULE DE BREVET EUROPEEN

(11) **EP 0 533 897 B1**
(45) Date de publication et mention de la délivrance du brevet: **13.03.1996**
(21) Numéro de dépôt: 92909585.9
(22) Date de dépôt: 30.03.1992
(51) Int. Cl.: A61B 17/115

(54) **AGRAFEUSE CHIRURGICALE**
Chirurgisches Klammernähgerät
SURGICAL STAPLER

(30) Priorité: 29.03.1991 FR 9103913
(43) Date de publication de la demande: 31.03.1993
(73) Titulaire: LABORATOIRE PEROUSE IMPLANT, F-60540 Bornel (FR)
(72) Inventeur: RICHARD, Thierry, F-75013 Paris (FR); PEROUSE, Eric, F-95290 L'Isle-Adam (FR); ARNISSOLLE, Yves, F-69230 Saint-Genis-Laval (FR)
(74) Mandataire: Jacobson, Claude
(86) Numéro de dépôt international: FR9200287
(87) Numéro de publication internationale: WO9217117

(56) Documents cités:
- FR-A- 2 171 477
- GB-A- 2 038 226
- US-A- 3 519 187
- US-A- 4 304 236
- US-A- 4 872 874

## Description

L'invention concerne un dispositif d'agrafage chirurgical destiné à réunir deux conduits constitués par un vaisseau et une prothèse tubulaire de vaisseau, du type décrit dans le préambule de la revendication 1.

Le corps humain comporte plusieurs types de conduits organiques, comme par exemple les organes de l'appareil digestif ou de l'appareil circulatoire. Au cours d'une intervention chirurgicale, on put être amené à suturer ensemble deux parties de conduits organiques sectionnées, par exemple à la suite de l'ablation partielle du conduit.

Dans le cas des vaisseaux, qui sont difficiles à suturer, il est connu d'aboucher une prothèse tubulaire sur les bouts de vaisseaux. La paroi de la prothèse est plus souple que celle du vaisseau, ce qui permet des manipulations plus aisées. On peut ainsi réunir deux parties de vaisseaux en abouchant d'abord un bout de prothèse de vaisseau aux extrémités de chacun d'eux, puis en abouchant ensuite les deux bouts de prothèse de vaisseaux par des moyens connus, manuels ou mécaniques.

Toutefois, dans la technique actuelle, les sutures intéressant les bouts de vaisseaux doivent être effectuées manuellement par couture.

L'invention a pour but de permettre de réaliser de façon commode et sûre des sutures mécaniques au moyen d'agrafes sur des conduits organiques relativement rigides, tels que des vaisseaux sanguins.

A cet effet, l'invention a pour objet une agrafeuse chirurgicale du type précité, caractérisée en ce que la prothèse tubulaire de vaisseau est enfilée sur une portion de la partie interne de l'agrafeuse, et en ce que des pointes de retenue anti-recul de la prothèse font saillie sur cette partie interne.

Il est à noter que l'art antérieur décrit des agrafeuses chirurgicales à couronnes d'agrafes ayant des pointes orientées radialement (GB-A-2 038 226, FR-A-2 171 477), mais que ces agrafeuses connues ne permettent pas d'aboucher commodément un vaisseau et une prothèse de vaisseau à l'intérieur du corps humain.

L'invention va être décrite plus en détail ci-dessous en se référant aux dessins annexés, sur lesquels:
- la figure 1 est une vue en coupe longitudinale d'une agrafeuse selon l'invention ;
- la figure 2 est une vue d'ensemble de l'agrafeuse;
- la figure 3 est une vue partielle en perspective des supports en porte-à-faux portant l'enclume ;
- la figure 4 est une demi-vue en coupe longitudinale du porte-agrafes ;
- la figure 5 est une demi-vue d'un secteur d'enclume suivant la flèche 5 de la figure 4 ;
- la figure 6 est une vue partielle en perspective de la partie interne de l'agrafeuse montrant la disposition d'orifices d'éjection des agrafes ;
- la figure 7 est une vue en coupe selon la ligne 7-7 de la figure 4 ;
- la figure 8 est une vue en coupe selon la ligne 8-8 de la figure 4 ;
- la figure 9 est une demi-vue en coupe longitudinale d'une variante de réalisation du porte-agrafes;
- la figure 10 est une vue en coupe suivant la ligne 10-10 de la figure 9 ;
- la figure 11 est une vue en coupe suivant la ligne 11-11 de la figure 9 ;
- la figure 12 est une vue partielle en perspective de la partie interne de l'agrafeuse montrant la disposition d'orifices d'éjection des agrafes suivant une variante de l'invention ;
- la figure 13 est une vue de face d'un orifice d'éjection d'agrafe selon cette variante de réalisation de l'invention;
- la figure 14 est une vue éclatée d'un second mode de réalisation de l'agrafeuse selon l'invention;
- les figures 15 à 18 illustrent le fonctionnement de l'agrafeuse de la Figure 14;
- les figures 19 et 20 sont des demi-vues à plus grande échelle prises respectivement en coupe suivant les lignes 19-19 et 20-20 de la figure 18;
- la figure 21 représente à encore plus grande échelle, en coupe axiale, la partie d'extrémité distale de la même agrafeuse;
- la figure 22 est une vue en coupe longitudinale d'un troisième mode de réalisation de l'agrafeuse suivant l'invention; et
- la figure 23 est une vue analogue à plus grande échelle de la partie d'extrémité distale de l'agrafeuse de la figure 22.

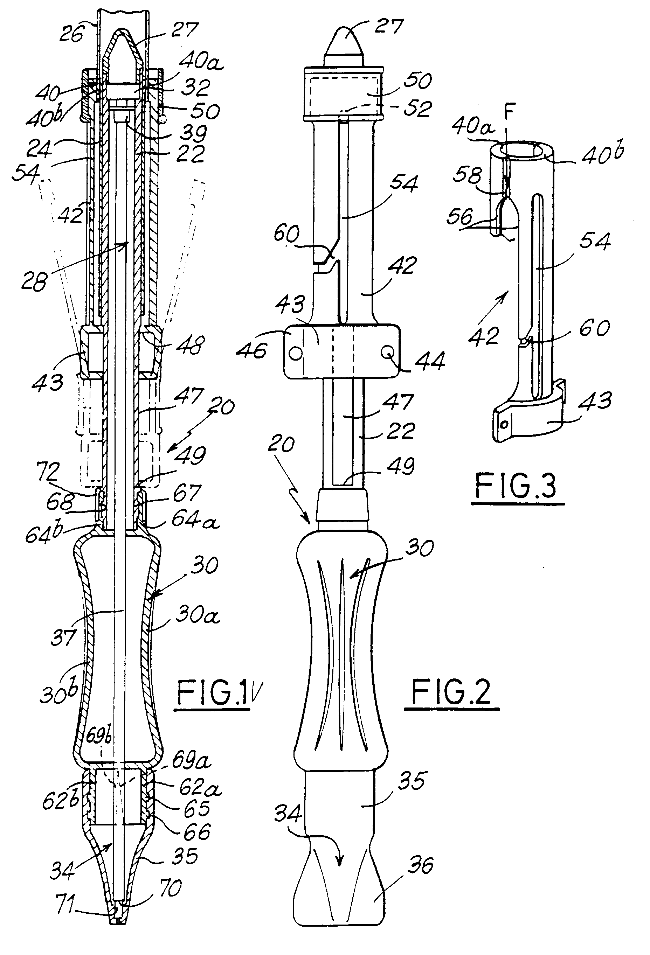

On voit aux figures 1 et 2, une agrafeuse 20 de forme générale allongée. Elle comporte un tube 22 dont l'extrémité distale est reçue dans des conduits, à savoir une prothèse 24 et un conduit organique 26. La prothèse 24 est enfilée sur la surface extérieure du tube 22 pour être agrafée avec le conduit organique 26, par exemple un vaisseau, recouvrant partiellement la prothèse 24 au niveau de l'extrémité distale du tube 22. L'extrémité distale du tube 22 comporte un capuchon 27, emmanché à force ou collé dans le tube, en forme d'ogive facilitant la pénétration de l'agrafeuse 20 dans les conduits. Le capuchon 27 et la partie du tube 22 autour duquel sont enfilés la prothèse 24 et le vaisseau 26 constituent une partie interne aux conduits 28 de l'agrafeuse. Le reste de l'agrafeuse constitue une partie externe aux conduits 29 de l'agrafeuse. La partie proximale du tube 22 est portée par une poignée d'agrafeuse 30 constituée de deux demi-poignées longitudinales 30a,30b.

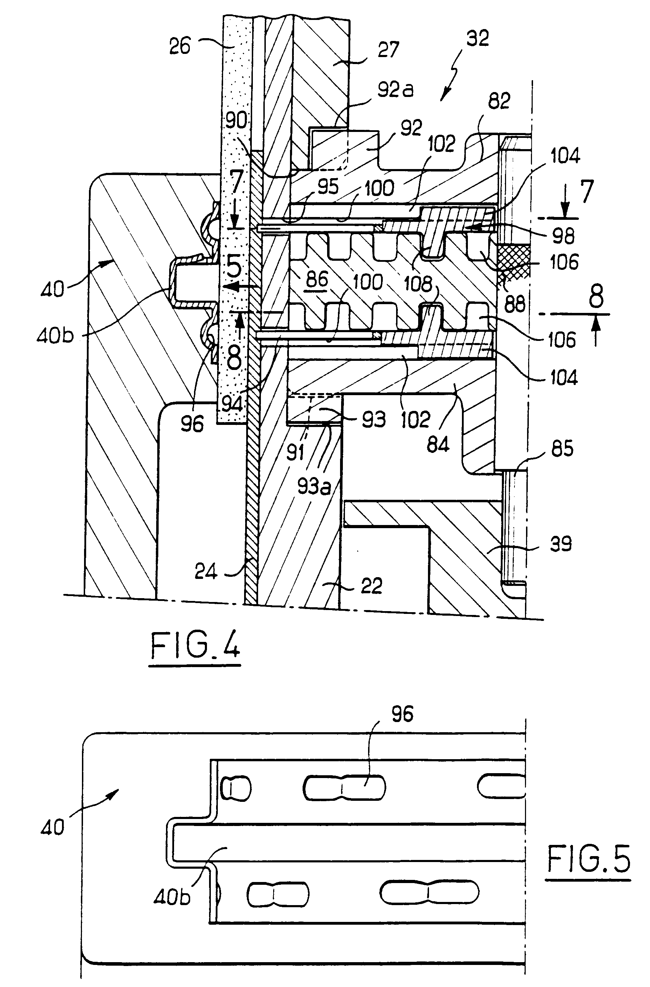

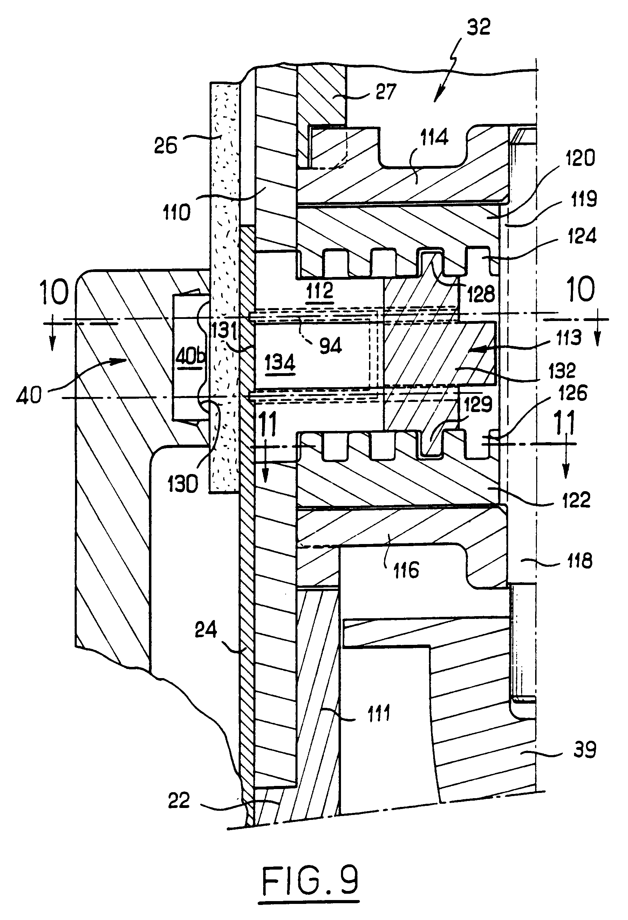

Un porte-agrafes 32, de forme générale cylindrique, est disposé à l'extrémité distale du tube 22, à l'intérieur de celui-ci. Il comporte des moyens d'éjection radiale des agrafes qui seront décrits ultérieurement et qui sont représentés en détail suivant un premier mode de réalisation du porte-agrafes à la figure 4 et suivant un deuxième mode de réalisation du porte-agrafes à la figure 9. Les moyens d'éjection des agrafes sont commandés par le manipulateur au moyen d'un papillon d'agrafage 34, monté rotatif sur l'extrémité proximale de la poignée 30. Le papillon 34 comporte un capuchon 35 muni de deux ailettes de manoeuvre 36. Il est relié aux moyens d'éjection des agrafes du porte-agrafes 32 par une tringlerie comprenant une tige 37, reliée au porte-agrafes 32 par une noix d'entraînement 39.

Une enclume 40 est disposée à l'extérieur du tube 22, au niveau du porte-agrafes 32, pour déformer les pointes des agrafes qui sont éjectées radialement du porte-agrafes 32. L'enclume 40 est montrée plus en détail aux figures 4 et 5 suivant un premier mode de réalisation et à la figure 9 suivant un second mode de réalisation. Dans les deux modes de réalisation, l'enclume 40 est constituée de deux secteurs d'enclume 40a,40b, portés chacun par un support 42 de forme générale semi-cylindrique, comportant un talon 43 formant une demi-bride, porté par le tube 22. Les deux demi-brides 43 sont assemblées l'une avec l'autre au moyen d'organes de liaison de type connu, par exemple des vis, disposées dans des trous 44 percés dans des ailes radiales 46 de chaque bride. Ces demi-brides 43 sont déplaçables axialement le long du tube 22 en glissant sur une portion de la surface extérieure de ce tube comportant un méplat 47 s'étendant longitudinalement et immobilisant en rotation les demibrides 43 par coopération de formes. Le déplacement longitudinal des demi-brides 43 est limité par deux épaulements 48,49 constituant les extrémités longitudinales de chaque méplat 47, et formant des butées de positionnement des supports 42 par rapport au porte-agrafes 32.

Les supports 42 sont faits par exemple dans un matériau métallique relativement élastique afin de pouvoir écarter à peu près radialement leurs extrémités distales portant les secteurs d'enclume 40a,40b afin de faciliter l'enfilage de la prothèse 24 et du vaisseau 26 sur le tube 22, entre ce dernier et les supports 42. Les supports 42 en position écartée sont montrés en traits mixtes à la figure 1.

Une bague de blocage 50 permet d'assembler les secteurs d'enclume entre-eux après avoir préalablement disposé les conduits organiques à agrafer sur la partie interne de l'agrafeuse et positionné les supports 42 en regard du porte-agrafes (demi-brides 43 en butée contre l'épaulement distal 48). La bague 50 est déplaçable axialement le long de la surface extérieure des supports 42. Un ergot 52 disposé radialement sur la paroi interne de la bague 50 coopère avec une rainure longitudinale 54 d'un des supports 42 pour guider la bague 50 entre les talons 43 formant des demi-brides et les extrémités distales des supports 42 portant les secteurs d'enclume 40a,40b. La bague 50 est faite dans un matériau relativement élastique, par exemple une matière plastique, de manière à pouvoir assembler les secteurs d'enclume 40a,40b par coincement de la bague autour des supports 42 au niveau des secteurs d'enclume 40a,40b.

On voit à la figure 3 une vue schématique des supports 42 permettant de comprendre le montage de la bague 50 sur ceux-ci. Les supports 42 sont d'abord montés sur l'agrafeuse et assemblés entre-eux par leurs talons 43 formant des demi-brides. On enfile ensuite la bague 50 autour d'eux en engageant l'ergot 52, suivant la flèche F, dans une rainure longitudinale 58 mitoyenne des deux supports 42. En déplaçant la bague 50 vers les talons 43 des supports 42 l'ergot est amené dans des évidements longitudinaux 56 des supports 42 qui sont en vis-à-vis. Finalement, on amène l'ergot 52 à hauteur d'une rainure 60 communiquant avec la rainure de guidage 54, ce qui permet de l'engager dans cette dernière.

On s'intéresse maintenant à la fixation et au montage de la poignée 30 et du papillon d'agrafage 34.

Les deux demi-poignées 30a,30b sont prolongées à leurs extrémités d'une part par des demi-manchons 62a,62b de support de papillon 34, et d'autre part par des demi- manchons 64a,64b de fixation sur le tube 22. Les deux demi-manchons 62a,62b de la poignée 30 comportent deux nervures annulaires 65 qui coopèrent avec des gorges annulaires 66 de forme complémentaire, de la paroi interne du papillon 34, en permettant la rotation libre du papillon 34 sur la poignée 30. L'extrémité proximale du tube 22 qui est emboîtée dans les demi-manchons 64a,64b de la poignée 30 comporte une saillie annulaire 67 de positionnement axial de la poignée 30 qui coopère avec une rainure annulaire 68 de forme complémentaire agencée sur les faces internes des demi-manchons 64a,64b.

Pour le montage de la poignée 30, on dispose tout d'abord le papillon d'agrafage 34 dans un support. On emboîte ensuite les demi-manchons 62a,62b de support de papillon dans le capuchon 35 du papillon 34. Ceci est obtenu en juxtaposant les bords de jonction 69a,69b des demi-manchons 62a,62b qui sont symétriquement tronqués, ce qui a pour effet de former un angle entre les deux demi-poignées 30a,30b. On engage l'extrémité proximale 70 de la tige 37, de section carrée, dans un trou 71 de forme complémentaire du capuchon 35 du papillon 34. On dispose l'extrémité proximale du tube 22, sur lequel on a préalablement enfilé une bague de sertissage coulissante 72, entre les deux demi-manchons 64a,64b de la poignée 30. On referme les demi-poignées 30a,30b l'une contre l'autre et on fait coulisser la bague de sertissage 72 sur la surface extérieure des demi-manchons 64a,64b en la coinçant sur ceux-ci.

Les figures 4 à 8 montrent un premier mode de réalisation du porte-agrafes 32 et de l'enclume 40 correspondante. Sur la figure 4, le porte-agrafes 32 et l'enclume 40 sont disposés verticalement. Le porte-agrafes 32 est constitué de deux flasques 82,84 solidaires du tube 22, portant une broche rotative 85 sur laquelle est fixé un disque 86 pouvant tourner entre les flasques 82,84. Le disque 86 est emmanché à force sur la zone médiane de la broche 85 qui comporte une portion de surface irrégulière 88 assurant une meilleure liaison entre le disque 86 et la broche 85. La broche 85 est reliée par une de ses extrémités de section carrée à la noix d'entraînement 39.

Le flasque 82 est positionné axialement à l'intérieur du tube 22 entre le bord 90 délimitant l'ouverture du capuchon 27 et le disque 86. Le flasque 84 est positionné axialement à l'intérieur du tube 22 entre un épaulement 91 correspondant à l'élargissement du chambrage intérieur du tube 22 et le disque 86. Les flasques 82,84 sont positionnés angulairement par rapport au tube 22, au moyen de saillies axiales 92,93 qui coopèrent avec des encoches de forme complémentaire 92a,93a, agencées respectivement sur le bord délimitant l'ouverture du capuchon 27 et sur l'épaulement 91 du tube 22.

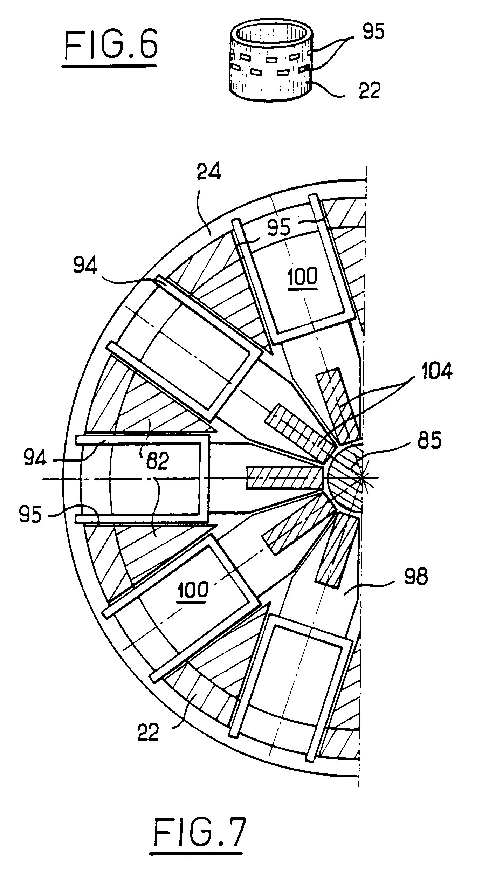

Des agrafes 94 sont disposées à plat entre les faces en regard du disque 86 et d'un flasque 82,84 de manière à constituer deux couronnes d'agrafes superposées. Chaque couronne d'agrafes 94 comporte dix agrafes réparties sur toute la circonférence du tube 22. Les agrafes 94 ont leurs pointes qui font saillie radialement à l'extérieur du tube 22 à travers des orifices 95 de celui-ci. A la figure 6, on voit comment les orifices 95 du tube 22 sont disposés suivant deux couronnes superposées, de telle façon que les orifices 95 d'une couronne soient en quinconce par rapport aux orifices 95 de l'autre couronne.

La figure 5 montre un secteur d'enclume 40b qui comporte des cavités 96, de forme connue, destinées à recevoir les pointes des agrafes pour les rabattre au moment de l'agrafage.

On décrit maintenant les moyens d'éjection des agrafes 94. Pour cela on se reporte aux figures 4,7 et 8. On voit aux figures 4 et 7 que chaque agrafe 94 est éjectée par un marteau 98, de forme générale plane. Les marteaux 98 sont disposés en couronnes entre les surfaces en regard du disque 86 et d'un flasque 82,84. Les flasques 82,84 comportent sur leur surface en regard avec le disque 86 des moyens de guidage des agrafes 94 et des moyens de guidage des marteaux 98. Les moyens de guidage des agrafes 94 sont constitués par des évidements 100 de la surface des flasques 82,84, s'étendant radialement dans lesquels sont logées les agrafes. Les moyens de guidage des marteaux 98 sont constitués par des rainures radiales 102 agencées sur la surface des flasques, au milieu des évidements 100, qui coopèrent avec une saillie de guidage 104 disposée sur une des faces des marteaux 98. Le disque 86 comporte des moyens d'entraînement des marteaux 98 constitués par une gorge en spirale 106, de pas constant, agencée symétriquement sur chaque face du disque. Les gorges en spirale 106 coopèrent avec chacune des saillies d'entraînement 108 disposée sur une des faces des marteaux 98.

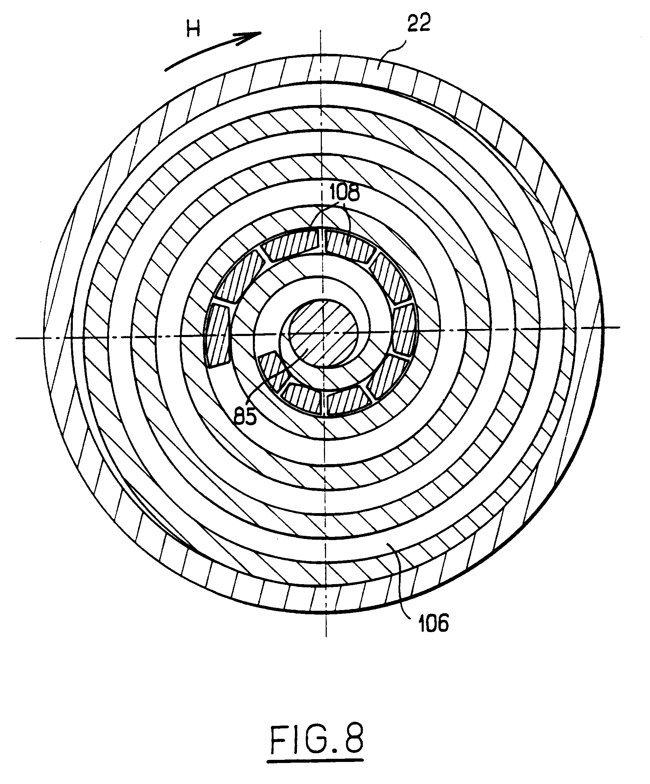

On voit à la figure 8 comment sont disposées les saillies d'entraînement 108 des marteaux 98 dans la nervure en spirale 106 du disque 86. Il y a dix saillies 108 ,correspondant à dix marteaux 98, qui éjectent simultanément une couronne de dix agrafes. Les marteaux 98 sont placés dans leur position initiale, avant agrafage, au centre du disque 86. La position de la saillie 108 sur chaque marteau 98 dépend de la position angulaire de celui-ci dans le porte-agrafes. Comme la gorge en spirale 106 est de pas constant, la rotation du disque dans le sens horaire, suivant la flèche H, entraîne simultanément tous les marteaux 98 en les déplaçant radialement suivant des distances identiques.

On décrit maintenant le montage du porte-agrafes 32 selon ce premier mode de réalisation. Dans un premier temps on place le capuchon 27 dans un support. Puis on assemble successivement le premier flasque 82, sur le capuchon 27, le disque 86 fixé sur la broche 85, et le deuxième flasque 84. On introduit ensuite les marteaux 98 dans le porte-agrafes 32. Comme la position de la saillie d'entraînement 108 sur chaque marteau 98 est fonction de la position angulaire de celui-ci dans le porte-agrafes 32, il convient de disposer les différents marteaux 98 dans le porte-agrafes 32 dans un ordre bien précis. Pour cela, on commence par introduire dans le porte-agrafes le premier marteau 98 de la série en engageant sa saillie d'entraînement 108 dans la gorge en spirale 106, par la périphérie du disque 86, au niveau d'un évidement 100 de guidage d'agrafes. On procède ensuite de la même façon avec les autres marteaux 98 de la série, en respectant l'ordre dans lequel ils sont disposés dans le porte-agrafes. Tous les marteaux 98 sont placés dans leur position initiale, au centre du disque 86, en tournant à fond le disque 86 dans le sens anti-horaire. On dispose alors l'ensemble capuchon 27-porte-agrafes 32 dans l'extrémité distale du tube 22. Les moyens de positionnement axial et de positionnement en rotation des flasques 82, 84 que l'on a décrit ci-dessus assurent la correspondance entre les orifices 95 d'éjection des agrafes du tube 22 et les évidements de guidage 100 des agrafes 94. Les agrafes 94 sont introduites dans le porte-agrafes 32 par les orifices 95 en orientant leurs pointes vers l'extérieur et en plaçant l'âme de chaque agrafe 94 au contact du marteau 98 correspondant. La longueur des marteaux 98 est telle que, lorsqu'ils sont en position initiale, les pointes des agrafes dépassent légèrement à l'extérieur du tube 22. Ainsi les pointes des agrafes 94 s'enfoncent légèrement dans la paroi de la prothèse 24, sans là traverser, de manière à la maintenir sur le tube 22.

On voit aux figures 9 à 11 un porte-agrafes 32 suivant un second mode de réalisation. Dans ce cas, celui-ci comporte une seule couronne d'agrafes 94 dont les âmes sont orientées axialement. A la différence du mode de réalisation précédent, le porte-agrafes 32 est disposé dans un manchon 110 qui est emmanché sur l'extrémité distale du tube 22, au niveau d'un rétrécissement 111 de la paroi extérieure du tube. Le manchon 110 comporte un collier 112, venu de matière, qui fait saillie à l'intérieur du manchon, et qui sert de support pour différents éléments du porte-agrafes 32. Comme pour le premier mode de réalisation, le porte-agrafes 32 comporte deux flasques 114,116 servant de support à une broche rotative 118 reliée à la noix d'entraînement 39 par coopération de formes. Dans le second mode de réalisation du porte-agrafes 32, le capuchon 27 est emmanché dans le manchon 110 et les flasques 114,116 sont positionnés axialement et angulairement par rapport au tube 22 de façon similaire au premier mode de réalisation du porte-agrafes 32. A la différence du premier mode de réalisation, deux disques d'entraînement 120,122 des marteaux 113, fixés sur la broche 118 par coopération de formes avec des cannelures axiales 119 de la broche, sont disposés de part et d'autre du collier 112. Ils portent chacun sur leur face en vis-à-vis avec une des faces du collier 112 une gorge en spirale 124,126, de pas constant, symétrique l'une de l'autre. Ces gorges d'entraînement 124,126 coopèrent avec deux saillies d'entraînement 128,129 de chaque marteau 113.

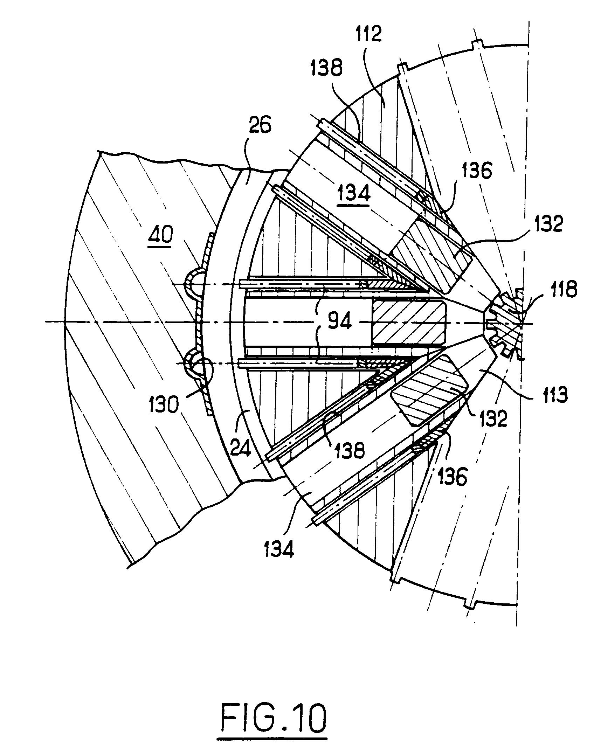

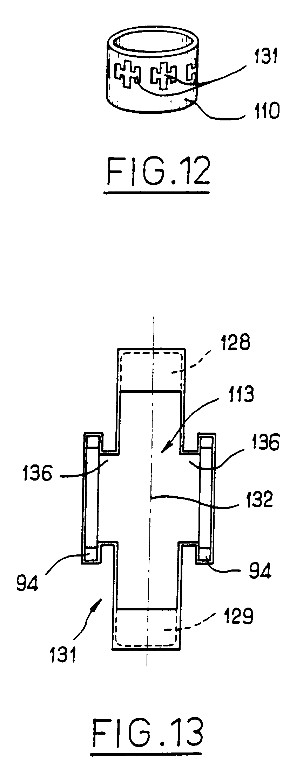

Sur la figure 9, le porte-agrafes 32 est disposé verticalement et les marteaux 113 sont dans leur position initiale avant l'agrafage, au centre du porte-agrafes 32. L'enclume 40 est dans ce cas adaptée pour recevoir des agrafes 94 dont les âmes sont orientées axialement par rapport au porte-agrafes. Elle est munie de cavités 130 de forme connue, destinées à replier les pointes des agrafes 94 au moment de l'agrafage. A la figure 10 on voit que le porte-agrafes 32 comporte dix marteaux 113 éjectant chacun deux agrafes 94 à travers des orifices 131. La figure 12 montre comment sont disposés les orifices 131 d'éjection des agrafes tout autour du manchon 110. La figure 13 montre un orifice 131 plus en détail. Celui-ci a une forme générale en croix. Les deux branches latérales de l'orifice en croix 131 ont des extrémités en forme de T, par chacune desquelles est éjectée une agrafe 94. De même, chaque marteau 113 a une forme générale en croix lorsqu'on le regarde face à un orifice 131 d'éjection des agrafes. Il est constitué d'un corps central 132 représenté verticalement aux figures 9 et 13. Le corps central 132 est guidé dans un trou de guidage radial 134 du collier 112. Le corps central 132 est muni en son milieu de deux branches latérales 136 dont les extrémités sont en forme de T comme le contour des orifices 131. Les extrémités des branches 136 de chaque marteau sont engagées dans des glissières 138 ménagées dans le collier 112 du manchon 110 et s'étendant radialement parallèlement aux trous de guidage 134 du corps central 132 des marteaux 113. Les agrafes 94 sont disposées dans les glissières 138 de telle façon que leur âme soit au contact des extrémités des branches 136 des marteaux 94.

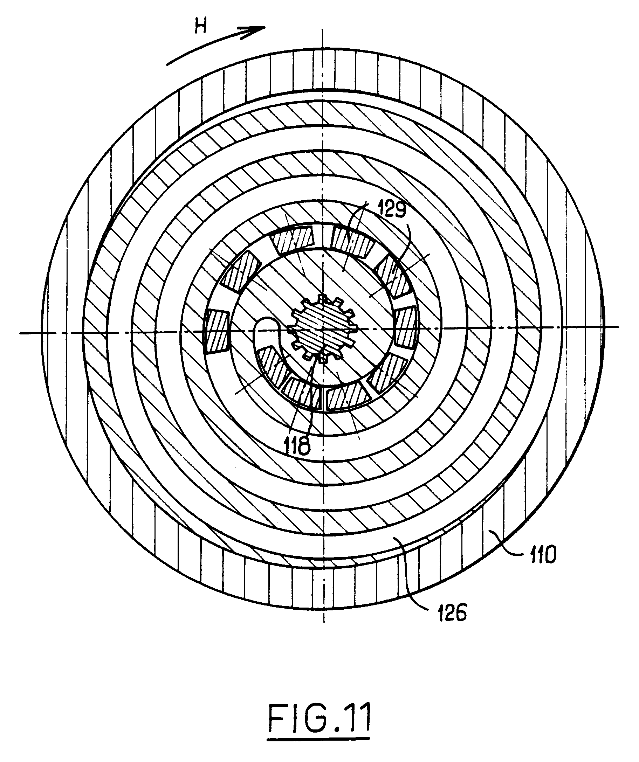

On voit à la figure 11 la gorge en spirale 126 dans laquelle sont reçues les saillies d'entraînement 129 des marteaux 113. On voit que la position des saillies 128,129 sur le corps central 132 des marteaux 113 dépend de la position angulaire du marteau 113 dans le porte-agrafes, comme pour le premier mode de réalisation du porte-agrafes.

Le montage du porte-agrafes 32 se fait suivant les principes généraux précédemment décrits dans le premier exemple de réalisation. On dispose le capuchon 27 dans un support puis on assemble successivement le flasque 114, la broche à cannelures 118, un premier disque d'entraînement 120, le manchon 110, un deuxième disque d'entraînement 122 et enfin le deuxième flasque 116. On dispose le manchon 110 et le porte-agrafes 32 sur l'extrémité distale du tube 22 et tout en faisant tourner les disques 120,122 dans le sens anti-horaire, on introduit les marteaux par les orifices 130 dans l'ordre. En dernier lieu on dispose les agrafes 94 dans les glissières 138 de part et d'autre des trous de guidage 134 des marteaux du manchon 110.

Comme dans le premier mode de réalisation du porte-agrafes, les pointes des agrafes 94 font saillie à l'extérieur de la partie interne 28 de l'agrafeuse pour s'enfoncer dans la paroi de la prothèse 24 sans la traverser, afin de la maintenir axialement sur l'agrafeuse.

Dans les deux modes de réalisation du porte-agrafes 32, l'agrafeuse est destinée à être utilisée avec une prothèse 24 préalablement mise en place sur le tube 22 à peu près entre les talons 43 des supports 42 et l'enclume 40. Pour mettre en place l'agrafeuse dans le vaisseau 26, on écarte les supports d'enclume 42 pour disposer le vaisseau 26 entre l'enclume 40 et le bout de la prothèse 24, au niveau du porte-agrafes 32. Les supports 42 sont ensuite positionnés axialement et les secteurs d'enclume sont assemblés entre-eux au moyen de la bague de blocage 50. Pour l'agrafage, le manipulateur saisit l'agrafeuse par la poignée 30 et fait tourner le papillon d'agrafage 34. La tige 37 entraîne en rotation la broche 85,118 par l'intermédiaire de la noix d'entraînement 39. Les marteaux 98,113 sont alors entraînés radialement vers la périphérie du tube 22 et éjectent les agrafes 94 contre l'enclume 40.

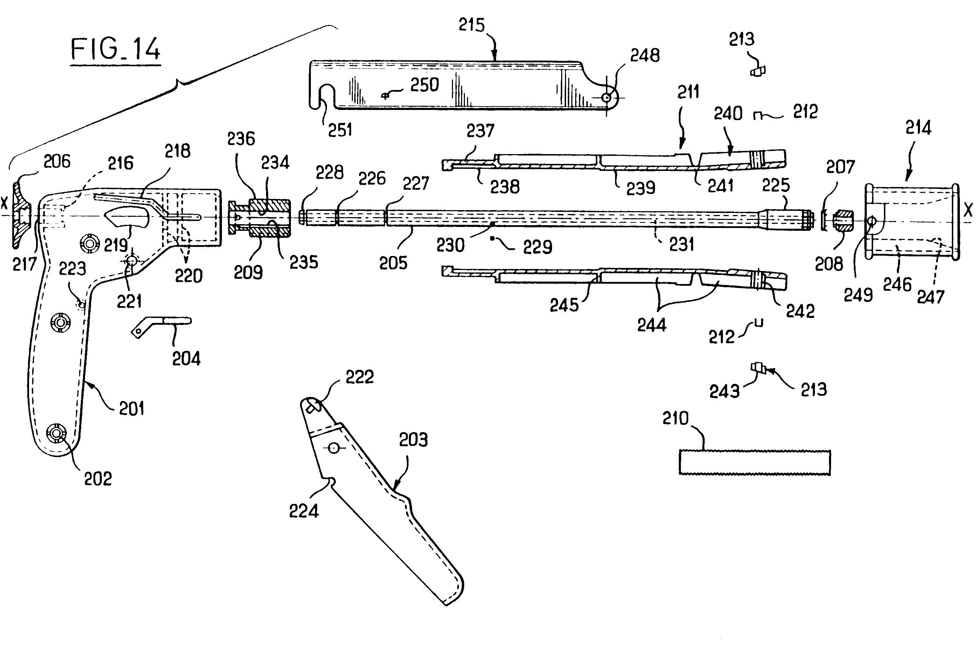

L'agrafeuse représentée aux figures 14 à 21 avec son axe X-X supposé horizontal est constituée des pièces suivantes, représentées sous forme éclatée à la figure 14 :
- une poignée 201, constitué de deux moitiés assemblées le long du plan de symétrie de l'agrafeuse par des vis 202;
- une détente 203 et son loquet de sécurité 204;
- une tige centrale creuse 205 et son bouton de manoeuvre annulaire 206;
- une bague de retenue 207 et un nez annulaire 208;
- une bague de montage 209;
- une prothèse tubulaire souple 210 en matière synthétique biocompatible, légèrement plissée en accordéon;
- six mâchoires ou secteurs 211;
- dans chaque mâchoire, deux agrafes 212 et un poussoir d'agrafe 213 commun pour ces deux agrafes;
- une bague 214 de fermeture des mâchoires et un levier d'armement 215.

Chaque moitié de la poignée 201 comporte intérieurement, à l'arrière, une pince élastique 216 entourant un orifice 217, et, dans sa partie avant, une fente de guidage 218, une fenêtre arquée 219 et des nervures verticales 220 de positionnement. La fente 218 comporte, de l'arrière vers l'avant, une partie légèrement descendante, une partie fortement descendante et une partie horizontale.

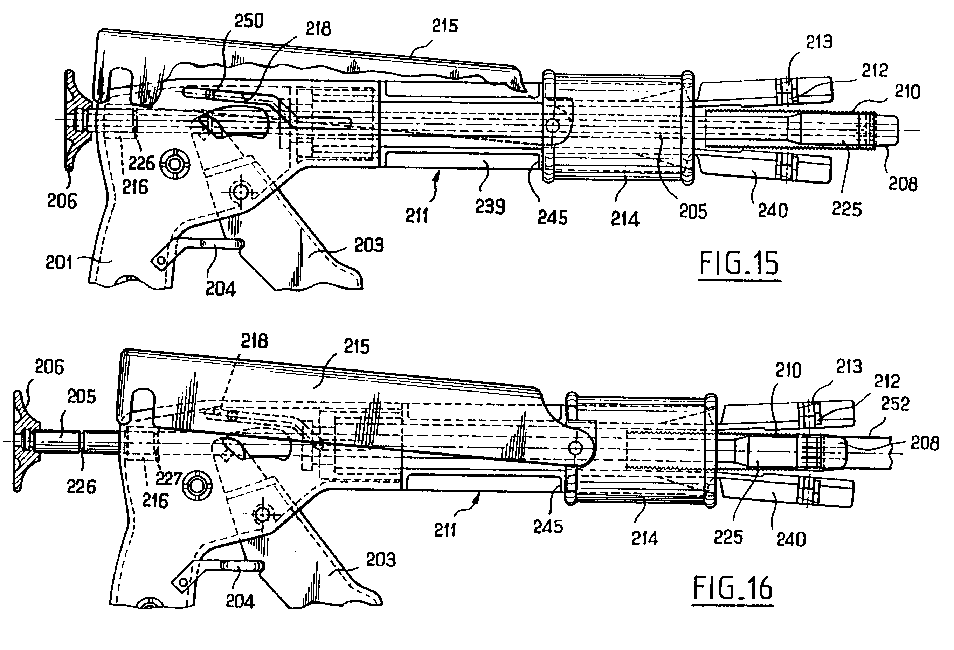

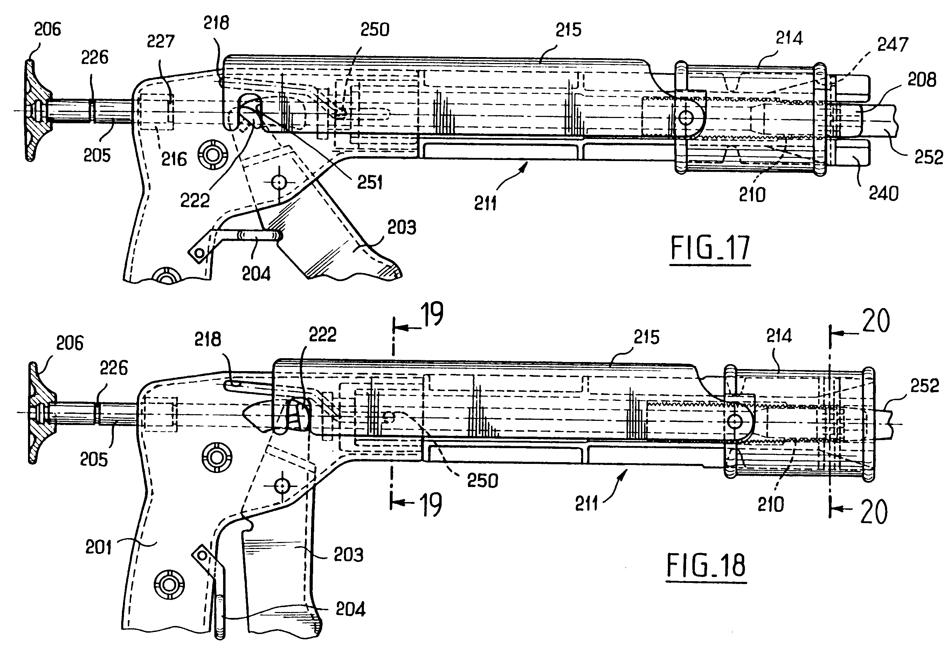

La détente 203 s'articule dans la poignée autour d'un axe 221. Elle présente à son extrémité supérieure une forme en T dont les branches 222 font saillie à travers les fenêtres 219. Le loquet 204 s'articule dans la poignée autour d'un axe 223 entre une position active (figures 15 à 17), à peu près horizontale, dans laquelle il pénètre dans une encoche 224 de la détente pour la bloquer en position inactive, et une position escamotée, à peu près verticale (figure 18), qui autorise l'actionnement de la détente.

La tige 205 a un diamètre extérieur uniforme sur presque toute sa longueur, sauf dans sa partie avant ou distale 225, dont le diamètre est agrandi. Cette tige comporte dans sa partie arrière ou proximale deux gorges circulaires 226, 227 situées à une petite distance l'une de l'autre, et elle se termine vers l'arrière par un embout 228 sur lequel se fixe le bouton 206. Un pion cylindrique 229 est à moitié emboîté dans un orifice 230 de la tige 205, et celle-ci comporte sur toute sa longueur un canal axial 231. L'extrémité avant de la partie 225 constitue une enclume et présente deux couronnes d'évidements 232 orientés axialement.

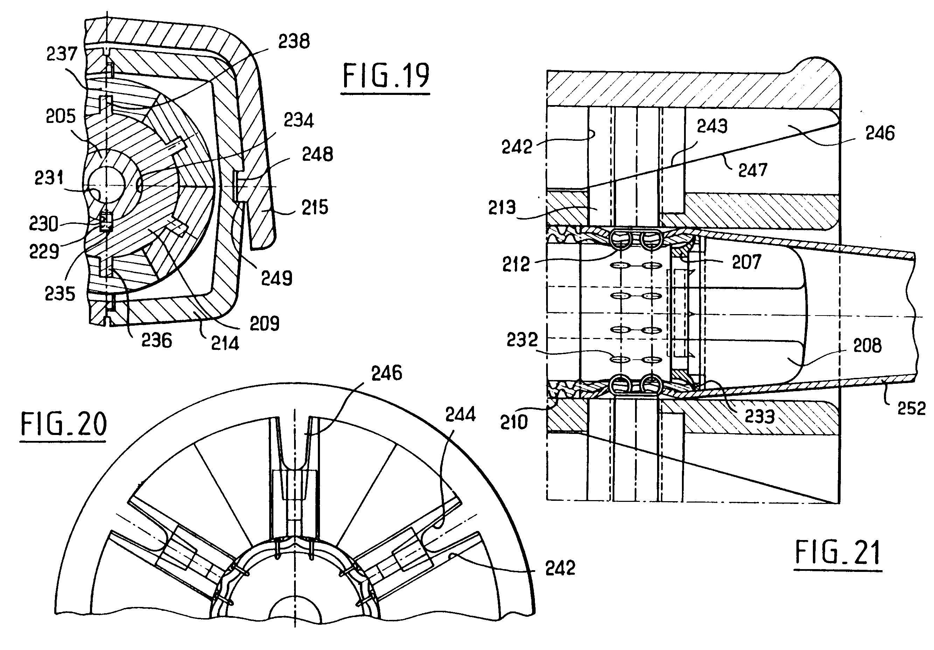

Comme on le voit mieux sur la figure 21, la bague de retenue 207 est un anneau plat fixé à l'extrémité distale de la tige 205 au moyen du nez 208. Ce dernier est extérieurement tronconique, convergent vers l'avant, tandis que la bague 207 comporte une couronne de pointes 233 orientées en oblique vers l'avant et en saillie radialement par rapport à l'enclume 225.

La bague de montage 209 est fixée dans la poignée au moyen des nervures 220. Elle comporte un alésage axial 234 traversé par la tige 205. La paroi de cet alésage comporte une rainure axiale 235 dans laquelle coulisse le pion 229 (figure 19), ce qui assure le positionnement angulaire de la tige 205 par rapport à la poignée. Axialement, cette tige peut être mise, au moyen du bouton 206, soit dans une position avancée (figure 15), dans laquelle le bouton est adjacent à la poignée, soit dans une position reculée (figures 16 à 18). Chacune de ces deux positions est définie avec précision par la venue élastique de la pince 216 dans la gorge 226 ou dans la gorge 227.

Extérieurement, dans sa partie avant, la bague 209 est pourvue de six nervures axiales 236 (figures 14 et 19).

Chaque mâchoire 211, réalisée en matière plastique, s'étend angulairement sur 60°, a la forme générale d'un secteur de cylindre et comporte trois parties venues de moulage :
- une partie arrière 237 délimitée entre deux épaulements radiaux internes et comportant une rainure axiale médiane 238 (figures 14 et 19), pour assurer son positionnement axial et angulaire sur la bague de montage 209;
- une partie intermédiaire 239 en porte-à-faux vers l'avant; et
- une partie avant 240 reliée à la précédente par une charnière déformable 241 et, au repos, légèrement divergente vers l'avant.

Dans la partie avant 240 de chaque mâchoire est ménagée une lumière radiale 242 dans laquelle est guidée à coulissement radial un poussoir d'agrafes 213, l'extrémité intérieure de cette lumière recevant deux agrafes parallèles 212. Les agrafes ont toute une âme orientée axialement et deux pointes dirigées à peu près vers l'axe X-X. Les poussoirs comportent une surface extérieure inclinée 243 formant rampe de came.

Chaque mâchoire présente de plus, le long des parties 239 et 240, une rainure axiale 244 qui coupe la lumière 242. Dans la partie 239, cette rainure est interrompue, à peu près à mi-longueur, par une nervure transversale 245.

La bague 214 présente intérieurement six nervures axiales 246 coulissant dans les rainures 244 respectives. Dans la partie avant de cette bague, les nervures ont une hauteur décroissante de façon à définir six rampes de came 247 de même inclinaison que les surfaces 243 des poussoirs d'agrafe.

Le levier d'armement 215 a une forme allongée à section en U (figure 19). A son extrémité avant, il comporte deux tétons intérieurs 248 (figures 14 et 19) reçus dans des évidements 249 diamétralement opposés prévus dans la bague 214. Dans sa partie arrière, le levier 215 comporte deux autres tétons 250 guidés dans les fentes 218 de la poignée, et, à son extrémité arrière, chaque branche du U présente une profonde échancrure 251.

L'utilisation de cette agrafeuse comporte quatre étapes, illustrées respectivement sur les figures 15 à 18.

Au départ, le levier 215 est reculé de manière à amener la bague 214 sur la partie 239 des six mâchoires 211, en butée arrière contre les nervures 245. Dans cette position, du fait de la forme inclinée vers l'avant des fentes 218, l'arrière du levier 215 est soulevé. De plus, le bouton 206 est enfoncé vers la poignée, avec la pince 216 en prise dans la gorge arrière 226 de la tige 205, de sorte que l'enclume 225 fait largement saillie vers l'avant au-delà des mâchoires, lesquelles sont ouvertes. Le chirurgien peut donc facilement observer l'introduction de l'enclume dans un bout de vaisseau sanguin 252 (figure 21) à raccorder à la prothèse 210, laquelle est enfilée sur l'enclume 225. Cette introduction est facilitée par la forme tronconique du nez 208, dont le diamètre maximal est sensiblement celui défini par les pointes 233. De préférence, dans le même but, la partie d'extrémité avant de la prothèse est lisse, c'est-à-dire non plissée. Pendant cette introduction, les pointes 233, qui pénètrent dans l'épaisseur de l'extrémité avant de la prothèse sans la traverser, empêchent tout recul de cette prothèse.

Lorsque l'enclume a suffisamment pénétré dans le vaisseau 252 pour que celui-ci recouvre la zone des deux couronnes d'évidements 232, le chirurgien, en retenant le bouton 206, pousse vers l'avant la poignée, et donc le reste de l'agrafeuse, jusqu'à ce que la pince 216 vienne en prise dans la gorge avant 227 de la tige 205. Il aboutit ainsi à la situation de la figure 16, qui ne diffère de celle de la figure 15 que par le fait que les évidements 232 de l'enclume se trouvent exactement au droit des pointes des douze agrafes 212 en attente dans les six mâchoires 211.

L'étape suivante (figure 17) consiste à pousser vers l'avant le levier d'armement 215, lequel pousse la bague 214 pour refermer les six mâchoires. Ce faisant, les parties inclinées des fentes 218 de la poignée obligent le levier 215 à se rabattre progressivement, jusqu'à ce que les deux échancrures 251 coiffent les bras en saillie 222 de la détente 203. L'extrémité avant de la bague 214 se trouve alors à une certaine distance en arrière de celle des mâchoires 211, avec les rampes 247 situées juste en regard des rampes 243 des poussoirs d'agrafe.

Il ne reste plus qu'à dégager le loquet 204 et à presser la détente. Les bras 222 poussent alors le levier 215, et donc la bague 214, vers l'avant, les deux tétons 250 étant alors guidés le long de la partie avant horizontale des lentes 218. La coopération des rampes 247 et 243 provoque le déplacement radial vers l'intérieur des six poussoirs 213, et donc l'éjection centripète simultanée des douze agrafes. Ces agrafes traversent le vaisseau et la prothèse et se recourbent dans les évidements 232. La position finale est celle représentée à la figure 21.

Pour retirer l'agrafeuse, on ramène vers l'arrière le levier d'armement 215. Le recul consécutif de la bague 214 libère les six mâchoires, qui s'ouvrent de nouveau par simple élasticité, et l'inclinaison vers l'avant des pointes 233 permet ensuite à l'enclume de sortir vers l'arrière de la zone agrafée puis de la prothèse, par simple traction sur la poignée 201.

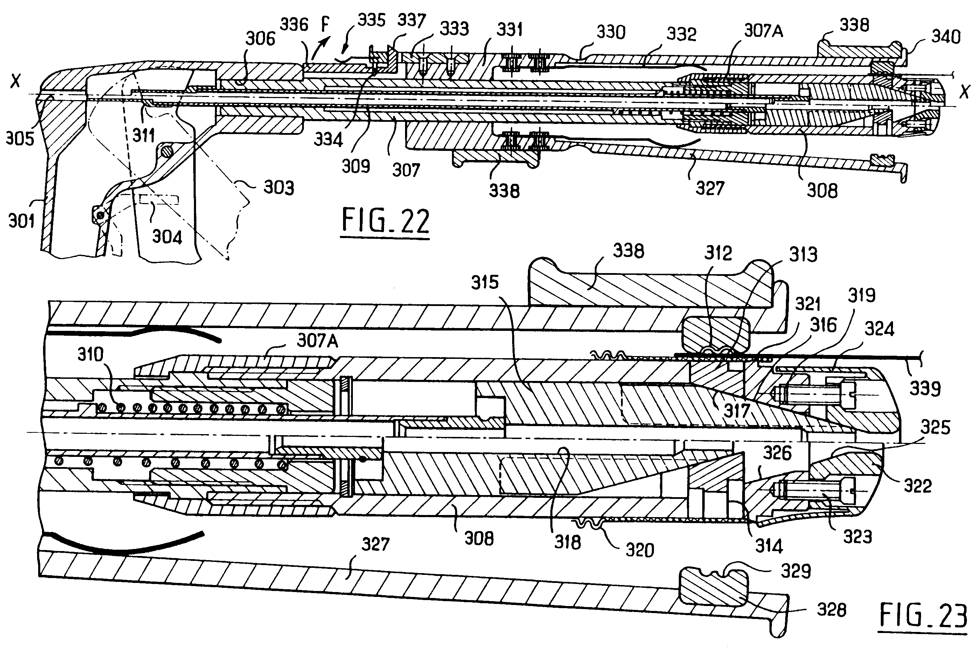

On décrira maintenant le mode de réalisation des figures 22 et 23, qui utilise également des cames coulissant axialement pour l'éjection des agrafes, mais qui est d'un type "centrifuge" comme dans le cas des figures 1 à 13.

On retrouve un ensemble poignée 301-détente 303-loquet 304, mais les ouvertures 218 et 219, les nervures 220 et la pince 216 du mode de réalisation précédent sont supprimées. La poignée présente sur l'axe X-X un orifice arrière 305 et un conduit avant 306 de plus grand diamètre dans lequel est fixé l'extrémité arrière, de diamètre réduit, d'un corps tubulaire principal 307. A l'extrémité avant de celui-ci est fixé, au moyen d'un écrou 307A, un corps avant tubulaire 308 de plus grand diamètre. Une tige creuse 309 coulisse dans l'ensemble 307-308 et est sollicitée vers l'arrière par un ressort hélicoïdal 310. Son extrémité arrière, à l'intérieur de la poignée, présente un gradin 311 qui s'appuie sur l'extrémité supérieure, fourchue, de la détente 303.

L'extrémité avant du corps 308 présente six lumières radiales 312 de guidage d'un poussoir d'agrafe 313 et de réception de deux agrafes parallèles 314 à âmes orientées axialement et à pointes dirigées à peu près radialement vers l'extérieur. Dans le corps 308 coulisse un éjecteur 315 ayant une partie arrière cylindrique et une partie avant tronconique. Cette partie avant définie une rampe de came 316 de même pente que la surface radialement intérieure 317 des poussoirs d'agrafe. L'éjecteur 315 comporte un canal central 318.

Une bague 319 de retenue de la prothèse 320 est fixée à l'extrémité avant du corps 308, et sa face arrière définit la face avant des lumières 312. Cette bague comporte une couronne de pointes 321 inclinées vers l'avant. La prothèse est enfilée sur le corps 308, et son extrémité avant, lisse, recouvre les lumières 312 et les pointes 321, qui pénètrent dans son épaisseur sans la traverser.

Un nez 322 est monté coulissant par rapport à la bague 319 au moyen de plusieurs vis axiales 323 vissées dans cette dernière. Ce nez comporte une jupe périphérique à fentes axiales définissant une série de languettes souples 324 dirigées vers l'arrière. Dans la position d'attente de l'agrafeuse, illustrée dans la moitié inférieure des figures 22 et 23, le nez est plaqué contre la bague 319, et son alésage central 325, convergent vers l'avant suivant la même pente que le cône 316, prolonge celui 326, de même pente, de la bague 319, tandis que l'extrémité arrière des languettes 324 recouvre l'extrémité avant de la prothèse.

L'agrafeuse comporte également six mâchoires 327 munies chacune à leur extrémité avant d'un secteur d'enclume 328 pourvu de deux évidements 329 orientés axialement. Chaque mâchoire est un secteur cylindrique en porte-à-faux raccordé par une mince charnière 330 à un support tubulaire arrière commun 331 qui coulisse sur le corps principal 307. Chaque secteur est sollicité vers une position d'ouverture par un ressort à lame 332.

Le support 331 est prolongé vers l'arrière par un appendice 333 d'où part vers l'axe X-X un ergot 334. Un frein de sécurité 335 en L est articulé sur cet ergot et comporte une branche 336 sollicitée par un ressort vers une position horizontale, et une branche 337 radiale. Cette dernière traverse vers l'extérieur une lumière de l'appendice 333 et se termine par une surface oblique inclinée vers le bas et vers l'avant.

Une bague de fermeture 338 peut coulisser sur l'ensemble des mâchoires 327, sur le support 331 et sur l'appendice 333.

Lorsque le support est dans une position arrière non représentée, avec la bague 338 en attente sur le support 331, le chirurgien voit la prothèse et le nez 322 et peut enfoncer celui-ci dans le vaisseau 339, dont le bout passe facilement sur la partie avant de la prothèse grâce aux languettes 324. Le chirurgien pousse alors vers l'avant le support 331. Lorsque celui-ci atteint une butée avant non représentée, les évidements 329 de l'enclume se trouvent exactement dans les plans des agrafes 314, et le frein 335 s'encliquette devant la face avant de la poignée 301 (figure 22). La situation est alors celle représentée dans la moitié inférieure des figures 22 et 23.

Puis le chirurgien pousse la bague 338 jusqu'à sa position extrême avant, définie par une butée d'extrémité 340 des mâchoires, ce qui ferme celles-ci.

Enfin, le chirurgien libère le loquet 304 et presse la détente 303. L'extrémité supérieure de celle-ci pousse vers l'avant la tige 309, en comprimant le ressort 310, et cette tige pousse l'éjecteur 315. Le cône 316, qui était juste en regard des surfaces 317 des six poussoirs, déplace ceux-ci radialement vers l'extérieur par effet de came, ce qui réalise l'agrafage simultané de la prothèse et du vaisseau par les douze agrafes.

Simultanément, l'extrémité avant du cône 316 vient au contact de l'alésage conique 325 du nez 322 et repousse celui-ci, jusqu'à ce que le cône bute contre l'alésage conique 326 de la bague 319. Comme on le voit dans la moitié supérieure des figures 22 et 23, ce déplacement du nez libère les languettes 324, qui reviennent élastiquement à une position paralléle à l'axe X-X.

Ceci permet de retirer l'agrafeuse vers l'arrière, mais il faut tout d'abord reculer le support 331, ce qui n'est possible que lorsque les mâchoires sont en position ouverte grâce à la présence du frein 335. En effet, il faut en premier lieu reculer à fond la bague 338. Lorsque celle-ci arrive sur l'appendice 333, elle escamote la branche radiale 337 du frein 335, et ce dernier bascule suivant la flèche f. Il est alors, et alors seulement, possible de reculer le support 331, avec par conséquent les mâchoires ouvertes, puis de retirer l'agrafeuse. On évite ainsi le risque de voir le chirurgien reculer par mégarde le support 331 alors que les mâchoires sont fermées, ce qui pourrait avoir pour effet d'arracher la zone agrafée du vaisseau 339.

On remarque que dans ce mode de réalisation comme dans celui des figures 14 à 21, l'agrafeuse comporte sur toute sa longueur un conduit toujours ouvert, ce qui permet le passage d'un cathéter ou d'un fluide.

## Revendications

1. Dispositif d'agrafage chirurgical pour réunir deux conduits constitués par un vaisseau (26; 252; 339) et une prothèse tubulaire de vaisseau (24; 210; 320), comportant d'une part une prothèse tubulaire de vaisseau (24; 210; 320), d'autre part une agrafeuse, ladite agrafeuse comprenant une première partie, dite partie interne, destinée à être reçue à l'intérieur des conduits, et une seconde partie, dite partie externe, demeurant à l'extérieur des conduits, l'une des deux parties de l'agrafeuse comprenant un porte-agrafes qui contient une série d'agrafes (94; 212; 314) disposées en au moins une couronne avec leurs pointes orientées radialement, et des moyens d'éjection de ces agrafes, l'autre partie de l'agrafeuse comprenant une enclume (40; 225; 328), l'agrafeuse comportant des moyens d'écartement radial relatif de l'enclume et du porte-agrafes par rapport à leurs positions relatives de travail, caractérisée en ce que la prothèse tubulaire (24; 210; 320) de vaisseau est enfilée sur une portion de la partie interne de l'agrafeuse, et en ce que des pointes (94; 233; 321) de retenue anti-recul de la prothèse font saillie sur cette partie interne.

2. Dispositif selon la revendication 1, caractérisé en ce que les pointes (94; 233; 321) attaquent la partie d'extrémité distale de la prothèse (24; 210; 320), laquelle partie d'extrémité est lisse.

3. Dispositif selon la revendication 1 ou 2, caractérisé en ce que les pointes (210; 320) sont solidaires de la partie interne de l'agrafeuse.

4. Dispositif selon la revendication 3, caractérisé en ce que les pointes (210; 320) sont inclinées vers l'avant.

5. Dispositif selon l'une quelconque des revendications 1 à 4, caractérisée en ce que la partie interne de l'agrafeuse comporte un nez (322) dont l'extrémité proximale présente un diamètre supérieur à celui de la prothèse (320) dans une position d'attente du nez et un diamètre inférieur à celui de cette prothèse lorsque l'éjection des agrafes (314) est réalisée.

6. Dispositif selon la revendication 5, caractérisée en ce que le nez (322) est monté coulissant axialement à l'extrémité de la partie interne de l'agrafeuse et comporte une série de languettes radialement élastiques (324) qui recouvrent l'extrémité distale de la prothèse (320) dans la position d'attente du nez, la partie interne de l'agrafeuse comprenant des moyens (316) pour pousser le nez vers l'avant à peu près au moment où l'éjection des agrafes est réalisée.

7. Dispositif selon l'une quelconque des revendications 1 à 6, caractérisée en ce que la partie externe comprend au moins deux secteurs (40a, 40b), et de préférence au moins trois secteurs (211; 327), déplaçables à peu prés radialement, et des moyens (50; 214; 338) d'assemblage des secteurs entre eux, notamment une bague coulissante, l'agrafeuse comprenant des moyens pour déplacer axialement un support de ces secteurs par rapport à la partie interne de l'agrafeuse en position écartée des secteurs.

8. Dispositif selon la revendication 7, caractérisée en ce que la partie externe comprend des moyens de butée qui définissent la position avant extrême des secteurs (40a, 40b; 327).

9. Dispositif selon la revendication 7 ou 8, caractérisée en ce qu'elle comprend un frein (335) pour le coulissement du support des secteurs (327), et des moyens de libération de ce frein actionnés par la mise en position inactive des moyens d'assemblage des secteurs (338).

10. Dispositif selon l'une quelconque des revendications précédentes, caractérisée en ce que les moyens d'éjection des agrafes (212; 314) comprennent des poussoirs d'agrafes ou marteaux (213; 313) guidés radialement, et des moyens d'entraînement des marteaux comprenant, pour chaque poussoir, une came guidée axialement (247; 316).

11. Dispositif selon l'une quelconque des revendications 1 à 10, caractérisée en ce que l'enclume (40; 328) est portée par la partie externe de l'agrafeuse.

12. Dispositif selon l'une quelconque des revendications 1 à 11, caractérisée en ce que toutes les agrafes (94; 212; 314) sont disposées sur une couronne d'agrafes, l'âme des agrafes étant orientée axialement.

13. Dispositif selon l'une quelconque des revendications 1 à 12, caractérisée en ce qu'elle comporte un canal central (231; 318) qui la traverse sur toute sa longueur.

14. Dispositif suivant la revendication 1 ou 2, caractérisé en ce que les pointes de retenue (94) sont constituées par les pointes des agrafes.

## Claims

1. Surgical stapler for connecting two canals consisting of a vessel (26; 252; 339) one a tubular vessel prosthesis (24; 210; 320), comprising on the one hand a tubular vessel prosthesis (24; 210; 320) and on the other hand a stapler, the said stapler comprising a first part, referred to as the internal part, intended to be received inside the canals, and a second part, referred to as the external part, remaining outside the canals, one of the two parts of the stapler comprising a staple holder which contains a series of staples (94; 212; 314) disposed in at least one ring with their tips oriented radially, and means for ejecting these staples, the other part of the stapler comprising an anvil (40; 225; 328), the stapler including means for the relative radial separation of the anvil and staple holder with respect to their relative working positions, characterised in that the tubular vessel prosthesis (24; 210; 320) is slipped onto a portion of the internal part or the stapler, and in that retaining points (94; 233; 321) for preventing backward movement of the prosthesis project on this internal part.

2. Device according to Claim 1, characterised in that the points (94; 233, 321) act on the distal end part of the prosthesis (24; 210; 320), which end part is smooth.

3. Device according to Claim 1 or 2, characterised in that the points (210; 320) are fixed to the internal part of the stapler.

4. Device according to claim 3, characterised in that the points (210; 320) are inclined towards the front.

5. Device according to any one of Claims 1 to 4, characterised in that the internal part of the stapler has a nose (322), the proximal end of which has a diameter greater than that of the prosthesis (320) in a standby position of the nose and a diameter less than that of this prosthesis when the staples (314) are ejected.

6. Device according to claim 5, characterised in that the nose (322) is mounted so as to slide axially at the end of the internal part of the stapler and has a series of radially elastic blades (324) which cover the distal end of the prosthesis (320) in the standby position of the nose, the internal part of the stapler comprising means (316) for pushing the nose forwards approximately at the time the staples are ejected.

7. Device according to any one of claims 1 to 6, characterised in that the external part comprises at least two sectors (40a, 40b), and preferably at least three sectors (211; 327), movable approximately radially, and means (50; 214; 338) for connecting the sectors together, notably a sliding ring, the stapler comprising means for axially moving a support for these sectors with respect to the internal part of the stapler in the separated position of the sectors.

8. Device according to claim 7, characterised in that the external part comprises stop means which define the extreme forward position of the sectors (40a, 40b; 327).

9. Device according to Claim 7 or 8, characterised in that it comprises a brake (335) for the sliding of the support for the sectors (327), and means for releasing this brake actuated by the putting of the means of connecting the sectors (338) into the inactive position.

10. Device according to any one of the preceding claims, characterised in that the means for ejecting the staples (212; 314) comprise staple pushers or hammers (213; 313) guided radially, and means for driving the hammers comprising, for each pusher, an axially guided cam (247; 316).

11. Device according to any one of Claims 1 to 10, characterised in that the anvil (40; 328) is carried by the external part of the stapler.

12. Device according to any one of claims 1 to 11, characterised in that all the staples (94; 212; 314) are disposed on a staple ring, the body of the staple being oriented axially.

13. Device according to any one of Claims 1 to 12, characterised in that it has a central channel (231; 318) which passes through it over its entire length.

14. Device according to Claim 1 or 2, characterised in that the retaining points (94) consist of the tips of the staples.

## Patentansprüche

1. Chirurgische Klammernahtvorrichtung zum Verbinden von zwei aus einem Gefäß (26; 252; 339) und einer rohrförmigen Gefäßprothese (24; 210; 320) bestehenden Leitungen, umfassend einerseits eine rohrförmige Gefäßprothese (24; 210; 320) und andererseits ein Klammernahtgerät, das einen ersten Teil, innerer Teil genannt, der dazu bestimmt ist, in das Innere der Leitungen einzutreten, und einen außerhalb der Leitungen bleibenden zweiten Teil, äußerer Teil genannt, besitzt, wobei einer der beiden Teile des Klammernahtgeräts einen Klammernträger, der eine Reihe von Klammern (94; 212; 314) enthält, die mit radial gerichteten Spitzen in mindestens einem Kranz angeordnet sind, und Mittel zum Ausstoßen dieser Klammern und der andere Teil des Klammernahtgeräts einen Amboß (40; 225; 328) aufweist, und das Klammernahtgerät Mittel besitzt, um den Amboß und den Klammernträger bezüglich ihrer relativen Arbeitsstellungen radial voneinander zu entfernen, dadurch gekennzeichnet, daß die rohrförmige Gefäßprothese (24; 210; 320) auf einen Abschnitt des inneren Teils des Klammernahtgeräts aufgesteckt ist und daß Spitzen zur Blockierung der Rückbewegung der Prothese auf diesem inneren Teil hervorstehen.

2. Vorrichtung nach Anspruch 1, dadurch gekennzeichnet, daß die Spitzen (94; 233; 321) an den distalen Endteil der Prothese (24; 210; 320), der glatt ist, angreifen.

3. Vorrichtung nach Anspruch 1 oder 2, dadurch gekennzeichnet, daß die Spitzen (210; 320) mit dem inneren Teil des Klammernahtgeräts fast verbunden sind.

4. Vorrichtung nach Anspruch 3, dadurch gekennzeichnet, daß die Spitzen (210; 320) nach vorne geneigt sind.

5. Vorrichtung nach einem der Ansprüche 1 bis 4, dadurch gekennzeichnet, daß der innere Teil des Klammernahtgeräts eine Nase (322) aufweist, deren proximales Ende einen Durchmesser hat, der in einer Wartestellung der Nase größer als der der Prothese (320) und, wenn der Ausstoß der Klammern (314) vorgenommen wird, kleiner als der dieser Prothese ist.

6. Vorrichtung nach Anspruch 5, dadurch gekennzeichnet, daß die Nase (322) am Ende des inneren Teils des Klammernahtgeräts axial verschiebbar montiert ist und eine Reihe von radial elastischen Zungen (324) aufweist, die in der Wartestellung der Nase das distale Ende der Prothese (320) überdecken, wobei der innere Teil des Klammernahtgeräts Mittel (316) aufweist, um die Nase ungefähr zum Zeitpunkt des Ausstoßens der Klammern nach vorne zu drücken.

7. Vorrichtung nach einem der Ansprüche 1 bis 6, dadurch gekennzeichnet, daß der äußere Teil mindestens zwei Sektoren (40a, 40b) und vorzugsweise mindestens drei Sektoren (211; 327), die ungefähr radial beweglich sind, und Mittel (50; 214; 338), insbesondere einen verschiebbaren Ring, zum Verbinden der Sektoren miteinander aufweist, wobei das Klammernahtgerät Mittel aufweist, um einen Träger dieser Sektoren bezüglich des inneren Teils des Klammernahtgeräts in der gespreizten Stellung dar Sektoren axial zu bewegen.

8. Vorrichtung nach Anspruch 7, dadurch gekennzeichnet, daß der äußere Teil Anschlagsmittel aufweist, die die vordere Endstellung der Sektoren (40a, 40b; 327) definieren.

9. Vorrichtung nach Anspruch 7 oder 8, dadurch gekennzeichnet, daß sie eine Bremse (335) für das Gleiten des Trägers der Sektoren (327) und Mittel zur Lösung dieser Bremse aufweist, die dadurch betätigt werden, daß die Mittel (338) zur Verbindung der Sektoren in die inaktive Stellung gebracht werden.

10. Vorrichtung nach einem der vorhergehenden Ansprüche, dadurch gekennzeichnet, daß die Mittel zum Ausstoßen der Klammern (212; 314) radial geführte Klammerndrücker oder Hämmer (213; 314) und Mittel zum Antrieb der Hämmer aufweisen, die für jeden Drücker einen axial geführten Nocken (247; 316) aufweisen.

11. Vorrichtung nach einem der Ansprüche 1 bis 10, dadurch gekennzeichnet, daß der Amboß (40; 328) von dem äußeren Teil des Klammernahtgeräts getragen ist.

12. Vorrichtung nach einem der Ansprüche 1 bis 11, dadurch gekennzeichnet, daß alle Klammern (94; 212; 314) auf einem Klammernkranz angeordnet sind, wobei der Mittelsteg der Klammern axial gerichtet ist.

13. Vorrichtung nach einem der Ansprüche 1 bis 12, dadurch gekennzeichnet, daß sie einen zentralen Kanal (231; 318) aufweist, der sie auf ihrer ganzen Länge durchquert.

14. Vorrichtung nach Anspruch 1 oder 2, dadurch gekennzeichnet, daß die Blockierspitzen (94) von den Spitzen der Klammern gebildet sind.
